# EUROPEAN PATENT APPLICATION

(11) **EP 4 699 574 A1**
(43) Date of publication of application: **25.02.2026**
(21) Application number: 25196629.7
(22) Date of filing: 19.08.2025
(51) Int. Cl.: A61F 2/34, A61F 2/46

(54) **IMPLANT LATTICE STRUCTURE FOR BONE GRAFT APPLICATION AND BONE PREPARATION**

(30) Priority: 20.08.2024 US 202463684982 P
(71) Applicant: Howmedica Osteonics Corp., Mahwah, NJ 07430 (US)
(72) Inventor: COBBE, Kevin, Dublin (IE)
(74) Representative: Keller Schneider Patentanwaltsgesellschaft mbH

(57) **Abstract**

An implant trial is configured for placement onto a joint of a bone. The implant trial includes a central portion sized for receipt in a j oint. The implant trial also includes a peripheral portion extending from the central portion. The peripheral portion includes a first region and a second region separate from the first region. When the central portion is received in the joint, the peripheral portion is configured to be received over a bone surface adjacent to the joint such that at least part of a surface of the first region is flush with a prepared bone surface and at least part of a surface of the second region faces a void in the bone. The second region has a lattice structure defined by a plurality of openings sized to allow bone graft to be received therethrough.

## Description

### CROSS REFERENCE TO RELATED APPLICATION

This application claims priority to U.S. Provisional Pat. App. No. 63/684,982 filed Aug. 20, 2024, the entire contents of which are incorporated herein by reference.

### BACKGROUND

Joint arthroplasty procedures may include replacement of a natural joint with an artificial joint. These procedures generally involve resection of a bone and securement of an implant onto the resected bone. Prior to securement of the implant onto the underlying bone, the bone must be prepared so that the implant will properly fit on the bone. If an implant is poorly seated on the bone, it may suffer a higher wear rate than a well-seated implant. Moreover, poorly seated implants may cause patient discomfort and/or impingement limiting the artificial joint's range of motion.

The correct positioning of an implant relative to a bone may be complicated by the existence of underlying bone that is weakened in certain areas due to damage or disease, such as osteolysis and the like, such that the bone is not adequate to support fixation members used to secure the implant to the bone. Visual inspection may help distinguish regions of healthy bone from those of unhealthy bone so that an operator may utilize the healthy bone to secure the prosthesis thereto. However, existing surgical instruments and implants often obscure the underlying bone, making it difficult to determine whether the implant may be adequately fixed to bone prior to an attempted securement of the implant to the bone.

Additionally, when a procedure involves replacement of the acetabulum, existing acetabular cup prostheses may include an excessive number of screw holes to ensure that at least one or some of the screw holes will align with dense, healthy bone when the acetabular cup is properly positioned. However, this excessive quantity of screw holes, many of which are not utilized, takes away from a surface area that may be made porous for bony ingrowth.

Accordingly, there is a need for improved implant trials usable to enhance surgical outcomes.

### BRIEF SUMMARY

The present disclosure relates to trials for use in surgical procedures. While largely discussed in connection with use in acetabular implants, the present disclosure is also applicable for preparation of implant placement in other joints, such as the shoulder, for example. Moreover, it is envisioned that the principles of the present disclosure may be employed in any area of anatomy where there is as needed to plan for bone void filling or leveling of a bone surface through resection.

In one aspect of the present disclosure, an implant trial is configured for placement onto a bone. The implant trial has a central portion sized for receipt in a joint, and the central portion has an articulation surface. The implant trial also has a peripheral portion extending from the central portion, and the peripheral portion has a first region and a second region separate from the first region. When the central portion is received in the joint, the peripheral portion is received over a bone surface adjacent to the joint such that at least part of a surface of the first region is flush with a portion of the bone surface and at least part of a surface of the second region faces a void in the bone. The second region has a lattice structure defined by a plurality of openings sized to allow bone graft to be received therethrough.

In some examples, the plurality of openings are a second plurality of openings and the peripheral has a third region separate from the first and second regions, and the third region includes a first plurality of openings having a different size than the second plurality of openings.

In some examples, the openings of the first plurality of openings are smaller than the openings of the second plurality of openings. In some examples, a maximal opening size in the third region is less than a size of each opening of the second plurality of openings.

In some examples, the first plurality of openings are distributed throughout the third region and the second plurality of openings are distributed throughout the second region.

In some examples, the peripheral portion consists of the first region, the second region and the third region.

In some examples, the openings in the first plurality of openings each have a maximum dimension in a range from 10 mm to 15 mm. In some examples, the openings in the second plurality of openings each have a maximum dimension in a range from 15 mm to 20 mm.

In some examples, the central portion is cup-shaped for receipt in an acetabulum. In some examples, the central portion includes a second plurality of openings therethrough, and the second plurality of openings are configured such that when the central portion is received in the acetabulum, a surface of the acetabulum is visible through the second plurality of openings. In some examples, the second plurality of openings is defined by a lattice structure.

In another aspect of the present disclosure, an implant trial is configured for placement onto a bone. The implant trial has a central portion sized for receipt in a joint, and the central portion has an articulation surface. The implant trial has a peripheral portion extending from the central portion and including a first region and a second region separate from the first region. The peripheral portion is configured such that when the central portion is received in the joint, the peripheral portion is received over a bone surface adjacent to the joint such that at least part of a surface of the first region is flush with a prepared bone surface and at least part of a surface of the second region faces an irregular bone surface. The second region has a lattice structure defined by a plurality of openings, and the plurality of openings are sized to allow bone graft to be received therethrough or to allow for projections of bone to pass therethrough.

In some examples, the first region extends from the central portion along a portion of a perimeter of the peripheral portion. In some examples, the portion of the perimeter is a first portion, and wherein the second region extends from the central portion along a second portion of the perimeter that opposes the first portion of the perimeter.

In some examples, the plurality of openings are sized to receive bone graft therethrough.

In some examples, the plurality of openings are sized to receive bone spurs of the bone therethrough and to allow for cutting of the bone spurs while the implant trial is positioned on the bone.

In some examples, a subpart of the at least part of the surface of the second region is flush with a corresponding part of the irregular bone surface when the implant trial is received over the bone surface.

In another aspect of the present disclosure, an implant trial is configured for placement onto a bone. The implant trial has a central portion sized for receipt in a joint, and the central portion includes an articulation surface. The implant also has a peripheral portion extending from the central portion. The peripheral portion includes a first region, a second region, and a third region separate from each other. When the central portion is received in the joint, the peripheral portion is configured to be received over a bone surface adjacent to the joint such that at least part of a surface of the first region is flush with a portion of the bone surface. The second region is defined by a first plurality of openings and the third region is defined by a second plurality of openings. At least a portion of the openings of the first plurality of openings are smaller than at least a portion of the openings of the second plurality of openings.

In some examples, the peripheral portion is a first peripheral portion, and the implant further includes a second peripheral portion opposing the first peripheral portion.

In some examples, the second peripheral portion includes a third plurality of openings.

In another aspect of the present disclosure, an implant trial is configured for placement onto a bone. The implant trial has a central portion sized for receipt in a joint, and the central portion has an articulation surface. The implant trial also has a peripheral portion extending from the central portion, and the peripheral portion has a first region and a second region separate from the first region. When the central portion is received in the joint, the peripheral portion is received over a bone surface adjacent to the joint such that at least part of a first surface of the first region is flush with a portion of the bone surface and at least part of a second surface of the second region faces a void in the bone. A first subportion of the first region encompassing the at least part of the first surface and a second subportion of the second region encompassing the at least part of the second surface have the same area in physical space. A side of the first subportion facing the bone has a different material surface area than a side of the second subportion facing the bone. The second region has a lattice structure defined by a plurality of openings sized to allow bone graft to be received therethrough.

In some examples, the plurality of openings are a second plurality of openings and the peripheral has a third region separate from the first and second regions, and the third region includes a first plurality of openings having a different size than the second plurality of openings.

In some examples, the openings of the first plurality of openings are smaller than the openings of the second plurality of openings. In some examples, a maximal opening size in the third region is different from a size of each opening of the second plurality of openings.

In some examples, the first plurality of openings are distributed throughout the third region and the second plurality of openings are distributed throughout the second region.

In some examples, the peripheral portion consists of the first region, the second region and the third region.

In some examples, the openings in the first plurality of openings each have a maximum dimension in a range from 10 mm to 15 mm. In some examples, the openings in the second plurality of openings each have a maximum dimension in a range from 15 mm to 20 mm.

In some examples, the central portion is cup-shaped for receipt in an acetabulum. In some examples, the central portion includes a second plurality of openings therethrough, and the second plurality of openings are configured such that when the central portion is received in the acetabulum, a surface of the acetabulum is visible through the second plurality of openings. In some examples, the second plurality of openings is defined by a lattice structure.

In another aspect of the present disclosure, an implant trial is configured for placement onto a bone. The implant trial has a central portion sized for receipt in a joint, and the central portion has an articulation surface. The implant trial has a peripheral portion extending from the central portion and including a first region and a second region separate from the first region. The peripheral portion is configured such that when the central portion is received in the joint, the peripheral portion is received over a bone surface adjacent to the joint such that at least part of a surface of the first region is contoured to be flush with a prepared bone surface and at least part of a surface of the second region is contoured to be positionable over and face an irregular bone surface. The second region has a lattice structure defined by a plurality of openings, and the plurality of openings are sized to allow bone graft to be received therethrough or to allow for projections of bone to pass therethrough.

In some examples, the first region extends from the central portion along a portion of a perimeter of the peripheral portion. In some examples, the portion of the perimeter is a first portion, and wherein the second region extends from the central portion along a second portion of the perimeter that opposes the first portion of the perimeter.

In some examples, the plurality of openings are sized to receive bone graft therethrough.

In some examples, the plurality of openings are sized to receive bone spurs of the bone therethrough and to allow for cutting of the bone spurs while the implant trial is positioned on the bone.

In some examples, a subpart of the at least part of the surface of the second region is flush with a corresponding part of the irregular bone surface when the implant trial is received over the bone surface.

In another aspect of the present disclosure, a method of evaluating a bone of a patient in preparation for receiving an implant includes placing an implant trial having a central portion and a peripheral portion so that the central portion is received in a joint of the patient and the peripheral portion is received over a bone surface adjacent to the joint. The method also includes determining a bone surface revision based on a relationship between a bone-facing surface of the peripheral portion and the bone surface of the patient. The relationship indicates a surface portion for at least one of bone resection and receipt of bone graft.

In some examples, determining the bone surface revision includes identifying a volume of bone for at least one of the bone resection and the receipt of bone graft. In some examples, determining the bone surface revision includes identifying the surface portion such that a first surface subportion of the surface portion is to be resected and a second surface subportion of the surface portion is to receive bone graft.

In some examples, the placing step includes passing one or more openings in the peripheral portion over respective projections on the bone surface such that the projections protrude from the peripheral portion after completion of the placing step.

In some examples, the method includes resecting the bone surface based on the determination of the bone surface revision. In some examples, the method includes placing bone graft on the surface portion through one or more openings in the peripheral portion. In some examples, the method includes resecting the bone surface before the placing step.

In some examples, the placing step includes receiving the central portion in an acetabulum of the patient and receiving the peripheral portion over a bone surface adjacent to the acetabulum.

### BRIEF DESCRIPTION OF THE DRAWINGS

FIG. 1 is a side perspective view of a trial in accordance with an aspect of the disclosure.
FIG. 2 is an inner plan view of the trial in FIG. 1.
FIG. 3 is a side perspective view of the opposite side of the trial in FIG. 1.
FIG. 4 is a side perspective view of a trial in accordance with another aspect of the disclosure.
FIG. 5 is an inner plan view of the trial in FIG. 4.
FIG. 6 is a side perspective view of the opposite side of the trial in FIG. 4.
FIG. 7 is an inner plan view of an implant secured on a bone in accordance with an aspect of the disclosure.
FIG. 8 is a flow chart of a method of using an implant trial in accordance with an aspect of the disclosure.

### DETAILED DESCRIPTION

When referring to specific directions in the following discussion of certain implantable devices, it should be understood that such directions are described with regard to the implantable device's orientation and position during exemplary application to the human body. Thus, as used herein, the term "proximal" means close to the heart and the term "distal" means more distant from the heart. Also, as used herein, the terms "about," "generally" and "substantially" are intended to mean that slight deviations from absolute are included within the scope of the term so modified. Wherever possible, and for the sake of brevity, the same or like reference numbers will be used throughout the drawings to refer to the same or like features within a different series of numbers (*e.g*., 100-series, 200-series, etc.).

References to first, second, third, and the like elements should be understood to merely distinguish various components from each other rather than to limit the use of such elements. Such elements may include different numbering and ordering without departing from the scope of the present disclosure. For example, a first element may be referred to as a second element, and a second element may be referred to as a first element.

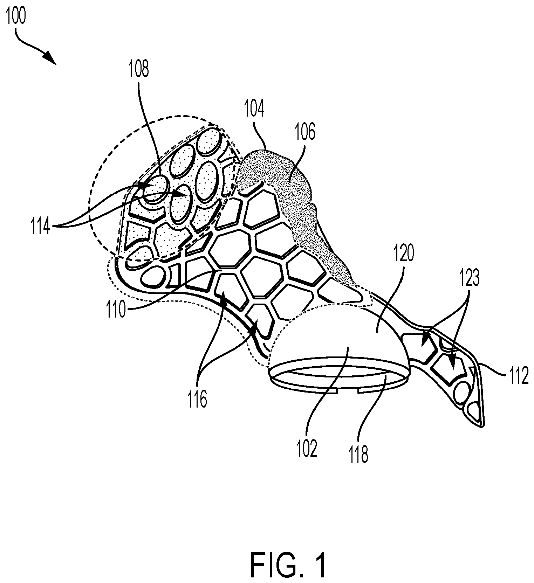

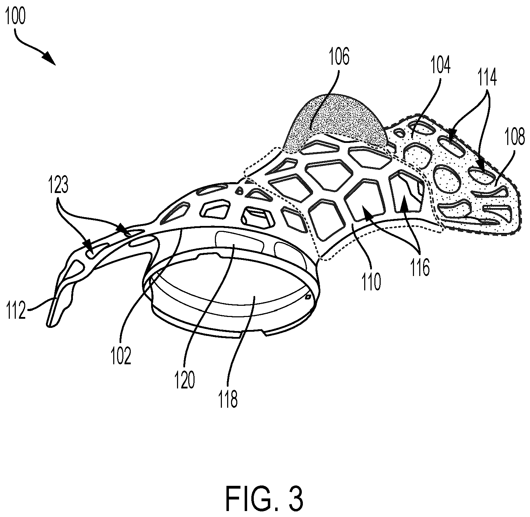

In one aspect, the present disclosure relates to an implant trial configured to aid in planning and preparing for securement of an implant in a patient as part of a surgical procedure. FIGS. 1-3 depict an implant trial 100 according to an example of the present disclosure. In this example, implant trial 100 is an acetabular implant trial used to plan and prepare for the placement of an acetabular cup implant in a patient. However, the implant trial may be shaped for use with any bone, such as a glenoid cavity. Implant trial 100 includes a central portion 102, a first peripheral portion 104 and a second peripheral portion 112. Each peripheral portion extends from central portion 102 and, as depicted, oppose each other. In variations, a relative location of first and second peripheral portions may be different. Further, in some variations, an implant trial may include one peripheral portion or three or more peripheral portions.

Central portion 102 is sized to be received in an acetabulum of the patient in a manner consistent with the acetabular cup implant to be secured in the patient. Central portion 102 is cup-shaped and includes a concave surface 118 and a convex surface 120, where convex surface 120 is bone-facing and is configured to be received on a bone. In some examples, and as shown, central portion 102 optionally includes an opening 122 at its apex for receiving an inserter instrument. For example, a guide rod may be inserted in opening 122 to direct the central portion 102 into an acetabulum. Opening 122 may be located at a same position on an inserter instrument as an implant, *e.g*., implant 300, when an implant is attached to an inserter instrument.

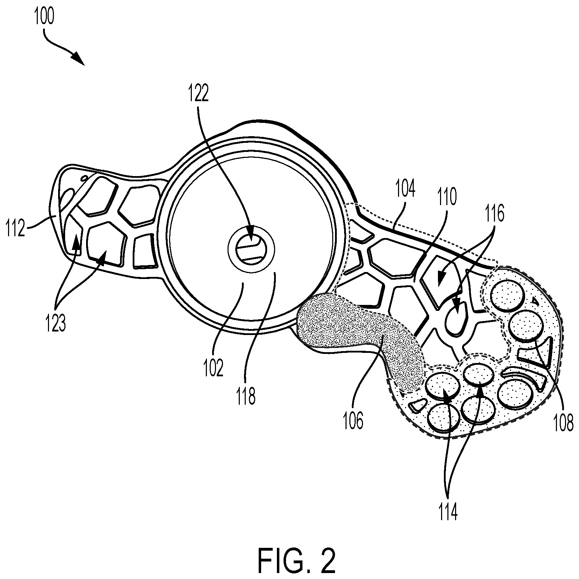

First peripheral portion 104 includes first, second, and third regions 106, 108, 110. In some examples, trial 100 may be arranged so that first peripheral portion 104 is configured to be placed on the illum. In first peripheral portion 104, first, second, and third regions 106, 108, 110 are distinct regions that each serve a primary function, as detailed below. However, in other examples, first, second, and third regions may partially or fully overlap or may otherwise vary in other ways. As shown in FIGs. 1 and 2, first and third regions 106, 110 extend from central portion 102 in a similar direction moving away from central portion. Second region 108 extends from first and third regions 106, 110 at an end farthest from central portion 102. However, in other examples, first, second, and third regions 106, 108, 110 may be arranged in any number of other ways on first peripheral portion 104.

When central portion 102 is received in the acetabulum of the patient, first region 106 is configured to be received over at least a portion of a bone surface adjacent to that joint. First region 106 has a bone-facing surface that is shaped to complement a portion of the bone surface so that when implant trial 100 is seated as intended on the joint of the patient, first region 106 is contoured to be flush against the portion of the bone surface. In some examples, first region 106 is configured to be flush against the illum. As depicted, first region 106 is solid without any openings of the type found in the other regions. However, in other examples, first region 106 may include openings with a size that is small relative to the openings in the other regions. In terms of function, additional surface area gained through the absence of openings concomitantly increases the surface area that may contact the bone surface.

Second region 108 includes a lattice structure defined by a first plurality of openings 114 with each opening passing entirely through a thickness dimension of first peripheral portion 104. As depicted, the openings of first plurality of openings 114 are circular in shape. In some examples, the openings may be polygonal in shape or have other shapes. The shape of first plurality of openings 114 allows a user to evaluate how many screws may or should be used and determine where holes for such screws should be located, *e.g.,* for determining screw hole locations in implant 300. Accordingly, the shape of first plurality of openings 114 may correspond to proposed screw locations once implant 300 is placed on the bone. Second region 108 is contoured to be positionable over and face an irregular bone surface, and first plurality of openings 114 are shaped and spaced apart so that when second region 108 is received on a target bone surface, the openings allow a user to assess bone quality and an area of bone that is present. Additionally, when implant trial 100 is placed on the bone surface, bone protrusions, such as bone spurs or osteophytes, may pass through the openings. This provides the user direction on where and how much bone resection should be performed. In some examples, each opening of first plurality of openings 114 may have a maximum dimension larger than a maximum dimension of a head on a screw intended to fix an implant to the bone. In some examples, the maximum dimension of each opening of first plurality of openings 114 is in a range from 6 mm to 20 mm. In other examples, the maximum dimension of each opening of first plurality of openings 114 is in a range from 10 mm to 15 mm. However, in other examples, the first plurality of openings may be any size that allows for receipt of bone protrusions and/or screw hole preparation.

Third region 110 includes a lattice structure defined by a second plurality of openings 116 with each opening passing entirely through a thickness dimension of first peripheral portion 104. As depicted, the openings of the second plurality of openings 116 are circular in shape. In some examples, the openings may be polygonal in shape so that the amount of material in the lattice is minimized, thus increasing visibility. In some examples, the openings may have other shapes. Third region 110 is configured to be positioned over a void in the bone surface when implant trial 100 is secured to an acetabulum. Further, second plurality of openings 116 are sized such that bone graft may be passed therethrough. Additionally, second plurality of openings 116 are sized so that a surface of the underlying bone is visible through second plurality of openings 116. In some examples, each opening of second plurality of openings 116 may have a maximum dimension in a range from 10 mm to 25 mm. In other examples, second plurality of openings 116 may each have a maximum dimension in a range from 15 mm to 20 mm. However, in other examples, second plurality of openings may be any size that allows for receipt of bone graft. Second plurality of openings 116 are a different size than first plurality of openings 114. Additionally, in some examples, all openings of first plurality of openings 114 may be smaller than all openings of second plurality of openings 116.

In the examples depicted in FIGs. 1-3, a unit area of a bone-facing surface of first region 106 has a different material surface area than a unit area of the same size in a bone-facing surface of second region 108, where the unit area is an area of the applicable surface portion in physical space. In the specific variation shown in FIGs. 1-3, a unit area of the bone facing surface of the first region 106 is solid throughout, so its material surface area is equal to the unit area, while a unit area (of the same size) in the second region 108 includes one or more openings, and thus has a lesser material surface area than the unit area in the first region. The referenced unit areas may also take the form of subportions of the respective first and second regions of the peripheral portion of the implant trial.

Similarly, a unit area of the bone-facing surface of first region 106 has a different material surface area than a unit area of the same size in the bone-facing surface of third region 110. In the specific variation shown in FIGs. 1-3, a unit area of the bone facing surface of the first region 106 is solid throughout, so its material surface area is equal to the unit area, while a unit area (of the same size) in the third region 110 includes one or more openings, and thus has a lesser material surface area than the unit area in the first region.

It should also be appreciated that material surface areas in respective equally sized unit areas of the second region 108 and third region 110 may also be different on account of those regions having openings of different sizes, i.e., a differing porosity between the two regions.

Second peripheral portion 112 is configured to be placed on a separate bone surface relative to first peripheral portion 104. In some examples, second peripheral portion 112 is shaped and oriented to be placed on the pubis. Second peripheral portion 112 includes a lattice structure defined by a third plurality of openings 123 with each opening in a direction perpendicular to the bone. As shown, third plurality of openings 123 may be used for a similar purpose as second plurality of openings 116, *i.e.,* to allow for passage of bone graft. Further, as with second and third regions 108, 110, the openings of third plurality of openings 123 render it possible to visualize the underlying bone surface.

In some examples, implant 100 may have a size such that its maximum diameter is in a range from 100 mm to 200 mm. However, implant 100 may have any maximum diameter as required for a specific patient's needs.

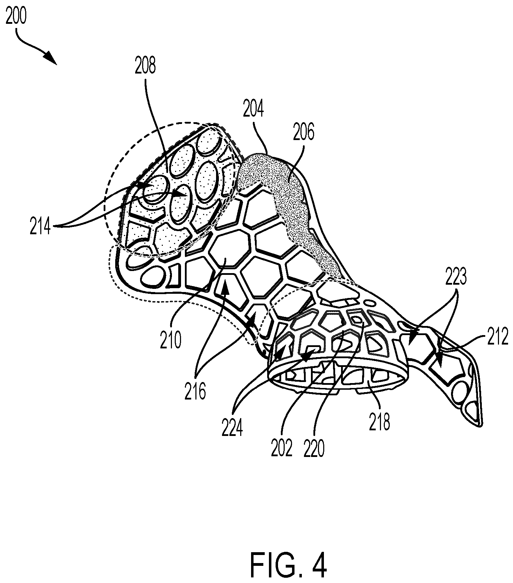

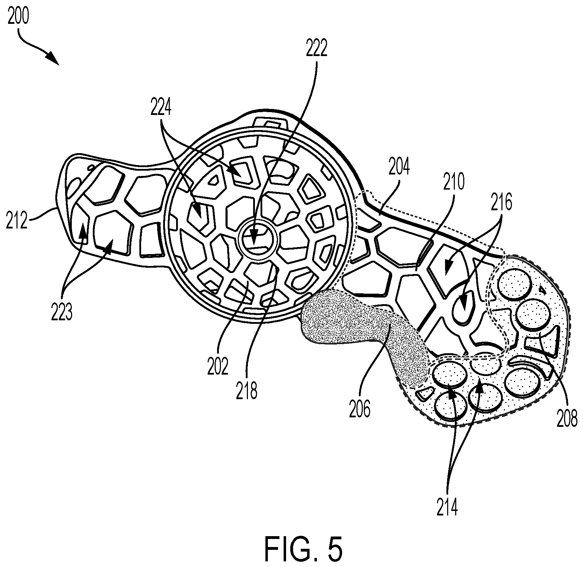

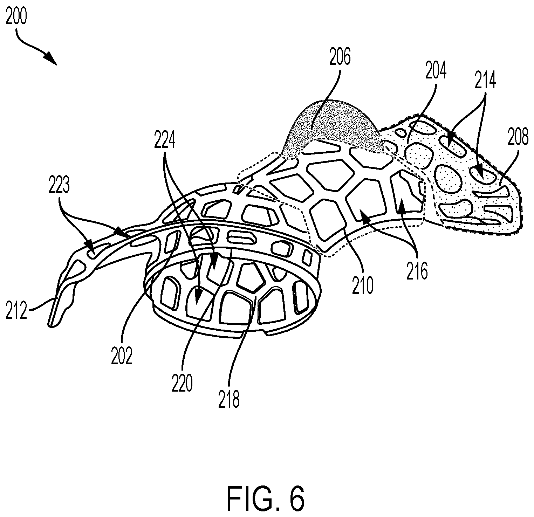

FIGS. 4-6 depict an implant trial 200 according to another example of the present disclosure. Implant trial 200 is similar to implant trial 100 of FIGs. 1-3, and therefore like elements are referred to with similar numerals within the 200-series of numbers. The description of certain similar features between implant trial 200 and implant trial 100 is omitted for sake of brevity, and the following description focuses on differences between implant trial 200 and implant trial 100.

Implant trial 200 differs from implant trial 100 in that central portion 202 includes a lattice structure defined by a fourth plurality of openings 224 with each opening passing through a thickness of the cup, *i.e.,* from the convex surface 220 to the concave surface 218. Fourth plurality of openings 224 are sized so that a surface of the joint received by the implant trial is visible through fourth plurality of openings 224 and so that a surface of the joint that receives the implant trial is accessible. Such access may be utilized for the receipt of bone graft, for example. Fourth plurality of openings 224 result in a decreased amount of material in central portion 202, resulting in a more economical and expeditious manufacturing process relative to fully solid trials.

In some alternative examples, fourth plurality of openings 224 of implant trial 200 may be arranged in any configuration that allows for receipt of bone graft. For example, central portion 202 may include a plurality of crescent-shaped viewing windows that extends from an apex of central portion 202 to an edge of central portion 202. Specifically, fourth plurality of openings 224 may converge into each other and taper to an end at their interface.

Implant trial 100, 200 may be made of any material suitable for implantation into a human body, including, but not limited to, polymeric materials (*e.g*., PEEK) and metallic materials (*e.g*., stainless steel or titanium). Implant trial 100,200 according to the present disclosure may be manufactured through additive layer manufacturing or via other methods that are known for use in fabricating implants and devices for placement into a body of a patient. Additive layer manufacturing includes, but is not limited to, three-dimensional printing, chemical etching, photo etching, laser cutting, water jet cutting, and traditional machining. Examples of additive layer manufacturing (ALM) techniques include electron beam melting, selective laser sintering (SLS), selective laser melting (SLM), and other three-dimensional (3-D) processes. When employing these technologies, articles are produced in layer-wise fashion from a laser-fusible powder that is dispensed one layer at a time. The powder is sintered in the case of SLS technology and melted in the case of SLM technology, by the application of laser energy that is directed in raster-scan fashion to portions of the powder layer corresponding to a cross section of the article. After the sintering or melting of the powder on one particular layer, an additional layer of powder is dispensed, and the process repeated, with sintering or melting taking place between the current layer and the previously laid layers until the article is complete. In one example, a high energy beam is emitted from a beam-generating apparatus to heat metal powder sufficiently to sinter and preferably to at least partially melt or fully melt the metal powder. High energy beam equipment for manufacturing such structures may be one of many commercially available. The beam generation equipment may also be a custom-produced laboratory device. Detailed descriptions of the SLS technology may be found in U.S. Pat. Nos. 4,863,538, 5,017,753, 5,076,869, and 4,944,817, the entire disclosures of which are incorporated by reference herein. Similarly, a detailed description of the use of SLM technology may be found in U.S. Pat. No. 7,537,664 ("the '664 patent"), the disclosure of which is incorporated by reference herein. The SLM and SLS technologies enable direct manufacture of solid or porous three-dimensional articles of high resolution and dimensional accuracy from a variety of materials including wax, metal and metal alloys, metal powders with binders, polycarbonate, nylon, other plastics and composite materials, such as polymer-coated metals and ceramics. Alternatively, the implant trial 100, 200 may be manufactured according to any of the methods disclosed in U.S. Pat. Nos. 8,268,099, 8,268,100, 8,992,703, 9,456,901, 10,182,923, 10,398,559, 10,525,688, 10,716,673, 10,835,388, 11,000,386, 11,155,073, and 11,622,867, the entire disclosures of which are hereby incorporated by reference herein.

Implant trial 100, 200 may include alignment markings (not shown) to be aligned with markings on the bone, particular anatomy, or general directions, such as superior and inferior. Alignment markings may assist with setting the alignment or rotational orientation of implant trial 100, 200 relative to the bone.

The implant trial may be varied in many ways. For example, an implant trial may be sized and shaped for any joint. Accordingly, a central portion may be sized and shaped for receipt in any joint, and any peripheral portions can be sized and shaped to complement any bone surface adjacent to the joint. For example, an implant may be sized and shaped for an acetabular cavity or a glenoid cavity.

An implant trial may include a single peripheral portion, two peripheral portions or three or more separate peripheral portions. In some examples, any one peripheral portion may include one or more of a plurality of openings for void filler, such as plurality of openings 116, a plurality of openings to allow passage of bone protrusions, such as plurality of openings 114, and solid-surface regions, such as first region 106 of first peripheral portion 104 in implant trial 100. Thus, in a subset of these examples, a peripheral region may be solid without any openings.

In some examples, first plurality of openings 114 may be arranged in any configuration and any shape that allows for receipt of bone protrusions. In some examples, second plurality of openings 116 may be arranged in any configuration and shape so that a surface of the underlying bone is visible through second plurality of openings 116.

In examples with two or more peripheral portions, a relative location of each peripheral portion may be any arrangement best suited to plan for implant securement. Similarly, in implant trials with any number of peripheral portions, a location of any peripheral portion relative to central portion may be any arrangement best suited to plan for implant securement.

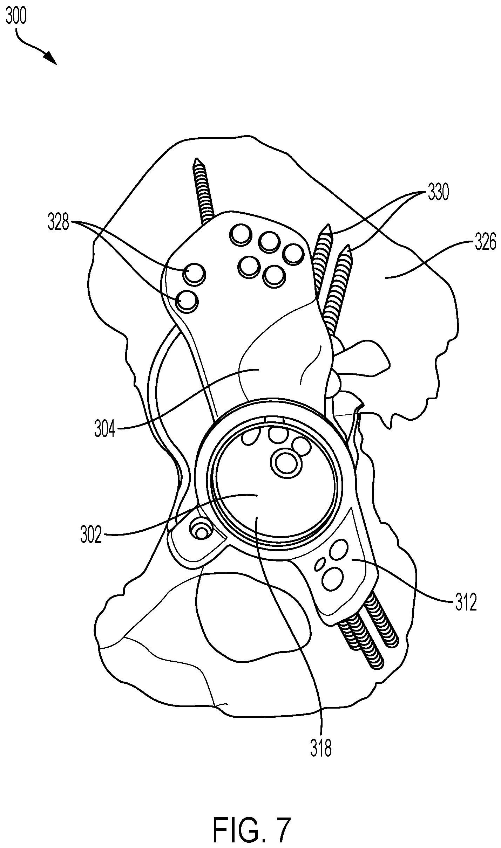

FIG. 7 depicts an implant 300 according to an example of the present disclosure. Implant 300 may complement implant trial 100, 200 such that use of the implant trial may prepare a bone surface for receipt of implant 300. In this example, implant 300 has geometry and peripheral dimensions commensurate with implant trial 100 of FIGs. 1-3 and as implant trial 200 of FIGs. 4-6. First and second peripheral portions 304, 312 have a plurality of screw holes 328 configured to receive screws 330 to secure implant 300 to the bone 326.

In another aspect, the present disclosure relates to a kit. for planning and preparing for securement of an implant in a patient. The kit may include one or more of an implant trial, an implant, and an instrument. Components of a kit may be any of those contemplated by the present disclosure. In one example, a kit may include an implant trial. In one example, a kit may include an implant trial and an implant, such as an acetabular implant trial and acetabular cup. In one example, a kit may include an implant trial, an implant, and an instrument. Further, in any one of the contemplated examples, a kit may include any number of accessories used with an instrument of the kit to for plan and prepare for securement of an implant in a patient. In one specific example, a kit may include implant trial 100, 200 and implant 300. In additional examples, a kit may include two or more implant trials, implants, and/or instruments. Contemplated kits that include two or more implant trials and/or implants may include implant trials and/or implants of varying sizes and/or shapes.

The kit may also include bone graft. Bone graft may be autologous and/or allogeneic bone graft, a bone growth enabling matrix, and/or bone growth stimulating substances. The bone growth enabling matrix may include endogenous bone forming cells (e.g., mesenchymal stem cells, osteoprogenitor cells, and osteoblasts) and osteoinductive and angiogenic growth factors. Additionally, the bone graft may be a solid substance such as a sol-gel bioactive glass (e.g., silicate, borate, and borosilicate bioglasses) or sol-gel derived bone graft.

The contents of the kit may be provided in a single package or in a series of packages. In some instances, each component of a kit may be provided in a separate package. A kit may further be provided with an instruction manual with details on how to use the contents of the kit. In some examples, instructions may be printed on the packaging itself. In any one of the examples that includes packaging, the packaging itself may include indicia and/or surfaces shaped to organize and/or label different components of a kit.

In another aspect, the present disclosure relates to a method of designing the implant trial. In some examples, a process of implant trial design begins with digitization of a patent scan, such as a CT scan. Such scan includes relevant anatomy of the patient, such as the pelvis when the implant trial is used in the acetabulum. With software, an operator may then evaluate the characteristics of the existing bone structure and model an implant trial design suited for the patient at issue. For example, when planning for implantation of a prosthesis in the acetabulum, areas of excess bone, such as bone spurs, osteophytes, and areas lacking sufficient bone, are identified and appropriate design features for peripheral portions of the implant trial are sized, positioned and shaped to account for those defects.

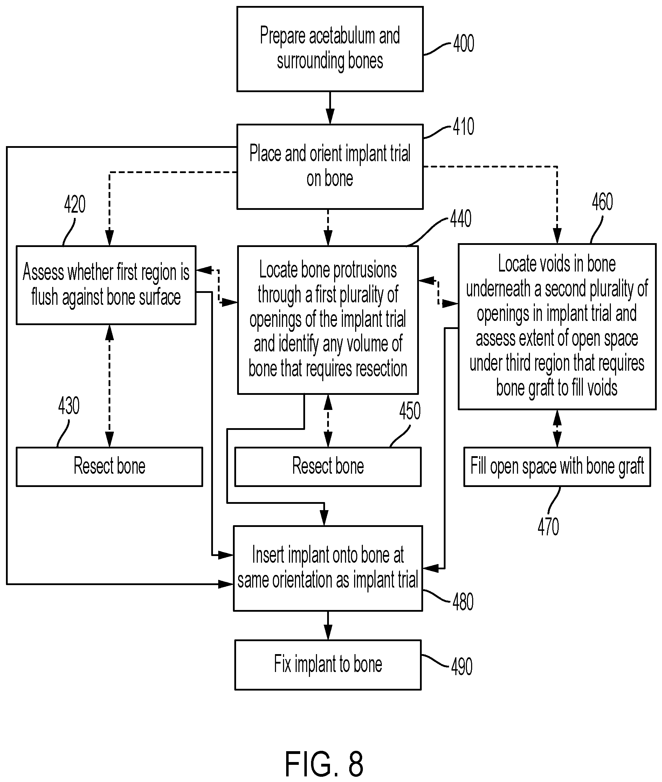

In yet another aspect, the present disclosure relates to a method of using an implant trial as part of a procedure to secure an implant within a patient. In one example of a method of using implant trial 100, 200, shown in FIG. 8, an operator gains access to a bone cavity that comprises a joint. In some examples, the joint may be a hip and the bone surface to be treated may be an acetabulum. The method may optionally include a step 400 in which the joint, such as the acetabulum, and surrounding bones are prepared by any method, such as with a reamer to resect the bone in preparation for receipt of an implant.

Once the bone is prepared and ready for trialing, a step 410 is performed in which the implant trial is placed on the bone. For example, when implant trial 100, 200 is used, convex surface 120, 220 of central portion 102, 202 contacts the bone within the acetabulum and peripheral portions 104, 112 are received over the bone surface adjacent to the acetabulum. Such placement may be aided with the use of a guide rod positioned on the acetabulum. In this regard, implant trial 100, 200 may be placed over the guide rod such that the guide rod extends through opening 122, 222. Then, as needed, implant trial 100, 200 is rotated so that peripheral portions 108, 112, 208, 212 are appropriately oriented relative to the bone. Optionally, such orientation may be aided by the use of alignment markings (not shown). The alignment markings may be aligned with markings on the bone, particular anatomy, or general directions, such as superior and inferior. Thus, the alignment markings may assist the operator with setting the alignment or rotational orientation of implant trial 100, 200 relative to the bone.

Once the implant trial is appropriately oriented, one or more of steps 420, 440 and 460 may be performed, as applicable based on the implant trial characteristics. The performance of these steps allows the operator to determine whether and to what extent the bone surface needs to be altered based on the fit of implant trial on the joint and bone surface adjacent to the joint. Specifically, when using implant trial 100, 200, the operator views the underlying bone through first and second plurality of openings 114, 116, 214, 216 to assess the bone quality beneath implant trial 100, 200 and to assess the fit of implant trial 100, 200 on the bone.

When step 420 is performed, the operator assesses whether a first region, *i.e.,* a region with a surface shaped to be flush with a complementary bone surface, is flush against the bone surface onto which the trial is placed. If the first region is not flush against the bone, a step 430 is performed in which operator further resects the bone until the first region is flush against the bone. When using implant trial 100, 200, step 420 includes assessing whether first region 106, 206 is flush against the illum on which it is placed, and, as applicable, step 430 includes further resection of the illum until first region 106, 206 is flush with the illum.

When step 440 is performed, the operator locates bone protrusions through a first plurality of openings in a second region of the implant trial and identifies any volume of bone that requires resection for an implant to fit flush against the bone surface under second region. Additionally, through the first plurality of openings, an operator may assess bone quality and consider an amount and location of screws. If the second region is not flush against the bone, a step 450 is performed in which operator further resects the bone until second region is flush against the bone. Bone spurs, osteophytes, and/or bone protrusions may be removed using instrumentation, including, but not limited to, osteotomes, burrs, or cobbs. Alternatively, an operator may use a surgical marker to indicate the bone spurs, osteophytes, and/or bone protrusions to be resected when the implant trial is removed. When using implant trial 100, 200, step 440 includes locating the bone protrusions through a first plurality of openings 114, 214 in a second region 108, 208 and identifying any volume of bone that requires resection for an implant to fit flush against the bone under second region 108, 208. As applicable, step 450 includes further resection of the bone until second region 108, 208 is flush with the bone and so that the operator has a relatively flat surface to drill into the bone, such as to drill holes in the bone to receive screws.

When step 460 is performed, the operator locates voids in the bone surface, assesses a volume of open space under a third region requires bone graft to fill the voids, and, in a step 470, fills that volume with bone graft. When using implant trial 100, 200, step 460 includes locating voids in the bone through a second plurality of openings 116, 216 and assessing a volume of open space under third region 110, 210 that requires bone graft to fill the void or voids. Additionally, as applicable, step 470 includes filling that volume with bone graft. Such filling may optionally be performed by inserting bone graft through one or more openings of second plurality of openings 116, 216. Once implant trial is removed from the bone, a step 480 is performed in which implant is inserted into the bone at the same orientation as the implant trial. Implant may then be fixed to bone in a step 490 using methods as known by persons of skill in the art. When using implant trial 100, 200, once implant trial 100, 200 is removed from the bone, step 480 includes inserting implant 300 in the same orientation as implant trial 100, 200. Step 490 may include drilling pilot holes into the bone through implant 300 and bone screws inserted therein to secure the implant 300 to the bone.

The performance of any or all of steps 420-470 allow for the preparation of a bone surface so that an implant may be in close contact with bone. For example, if an implant with a partially or fully porous surface is used, any or all of steps 420-470 allow for bone ingrowth through the pores due to the close contact between the implant and bone.

The method may be varied in many ways. In examples in which the implant trial includes a fourth plurality of openings in the central portion, an operator may view the joint through the fourth plurality of openings or access the joint through the fourth plurality of openings, such as to insert bone graft onto the joint through the fourth plurality of openings. When using implant trial 100, 200, an operator may view or access the acetabulum or insert bone graft onto the acetabulum through fourth plurality of holes 224.

Additionally, a complete method of using a trial implant may include any one or more of the above method steps up to and including each of the described steps. In other examples, any joint and surrounding bone may be prepared, and an implant trial may be placed on any joint. Additionally, the bone may be imaged before any of the steps described above, *e.g.,* by X-ray, CT scan, or any other imaging technique.

The disclosure set forth herein includes any possible combinations of the particular features set forth above, whether specifically disclosed herein or not. For example, where a particular feature is disclosed in the context of a particular aspect, arrangement, configuration, or arrangement, that feature can also be used, to the extent possible, in combination with and/or in the context of other particular aspects, arrangements, configurations, and arrangements of the technology, and in the technology generally.

Furthermore, although the technology herein has been described with reference to particular features, it is to be understood that these features are merely illustrative of the principles and applications of the present technology. It is therefore to be understood that numerous modifications, including changes in the sizes of the various features described herein, may be made to the illustrative arrangements and that other arrangements may be devised without departing from the spirit and scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. An implant trial (100, 200) configured for placement onto a bone, the implant trial (100, 200) comprising:
a central portion (102, 202) sized for receipt in a joint, the central portion (102, 202) including an articulation surface; and
a peripheral portion (104, 204) extending from the central portion (102, 202), the peripheral portion (104, 204) including a first region (106, 206) and a second region (108, 110, 208, 210) separate from the first region (106, 206),
wherein when the central portion (102, 202) is received in the joint, the peripheral portion (104, 204) is configured to be received over a bone surface adjacent to the joint such that at least part of a surface of the first region (106, 206) is flush with a portion of the bone surface and at least part of a surface of the second region (108, 110, 208, 210) faces a void in the bone, and
wherein the second region (108, 110, 208, 210) has a lattice structure defined by a plurality of openings (116, 216) sized to allow bone graft to be received therethrough.

2. The implant trial (100, 200) of claim 1, wherein the plurality of openings are a second plurality of openings (116, 216) and the peripheral portion (104, 204) further comprises a third region (108, 208) separate from the first and second regions (106, 206, 110, 210), the third region (108, 208) including a first plurality of openings (114, 214) having a different size than the second plurality of openings (116, 216).

3. The implant trial (100, 200) of claim 2, wherein the openings of the first plurality of openings (114, 214) are smaller than the openings of the second plurality of openings (116, 216).

4. The implant trial (100, 200) of claims 2 or 3, wherein a maximal opening size in the third region (108, 208) is less than a size of each opening of the second plurality of openings (116, 216).

5. The implant trial (100, 200) of claim 4, wherein the first plurality of openings (114, 214) are distributed throughout the third region (108, 208) and the second plurality of openings (116, 216) are distributed throughout the second region (110, 210).

6. The implant trial (100, 200) of claim 5, wherein the peripheral portion (104, 204) consists of the first region (106, 206), the second region (110, 210) and the third region (108, 208).

7. The implant trial (100, 200) of any of claims 2 to 6, wherein the openings in the first plurality of openings (114, 214) each have a maximum dimension in a range from 10 mm to 15 mm.

8. The implant trial (100, 200) of any of claims 2 to 7, wherein the openings in the second plurality of openings (116, 216) each have a maximum dimension in a range from 15 mm to 20 mm.

9. The implant trial (100, 200) of any preceding claim, wherein the central portion (102, 202) is cup-shaped for receipt in an acetabulum.

10. The implant trial (100, 200) of claim 9, wherein the central portion (102, 202) includes a second plurality of openings (116, 216) therethrough, the second plurality of openings (116, 216) being configured such that when the central portion (102, 202) is received in the acetabulum, a surface of the acetabulum is visible through the second plurality of openings (116, 216).

11. The implant trial (100, 200) of claim 1, wherein the plurality of openings are a second plurality of openings (116, 216)
and the peripheral portion (104, 204) further comprises a third region (108, 208) separate from the first and second regions (106, 206, 110, 210),
wherein the peripheral portion (104, 204) is configured such that when the central portion (102, 202) is received in the joint, the peripheral portion (104, 204) is received over a bone surface adjacent to the joint such that at least part of a surface of the third region (108, 208) faces an irregular bone surface, and
wherein the third region (108, 208) has a lattice structure defined by the first plurality of openings (114, 214), the first plurality of openings (114, 214) being sized to allow for projections of bone to pass therethrough.

12. The implant trial (100, 200) of claim 11, wherein the first region (106, 206) extends from the central portion (102, 202) along a portion of a perimeter of the peripheral portion (104, 204).

13. The implant trial (100, 200) of claim 12, wherein the portion of the perimeter is a first portion, and wherein the third region (110, 210) extends from the central portion (102, 202) along a second portion of the perimeter that opposes the first portion of the perimeter.

14. The implant trial (100, 200) of any of claims 11 to 13, wherein the first plurality of openings (114, 214) are sized to receive bone spurs of the bone therethrough and to allow for cutting of the bone spurs while the implant trial is positioned on the bone.

15. The implant trial (100, 200) of any of the preceding claims, wherein the first region (106, 206) is solid.
